# EUROPÄISCHE PATENTSCHRIFT

(11) **EP 2 727 519 B1**
(45) Veröffentlichungstag und Bekanntmachung des Hinweises auf die Patenterteilung: **14.08.2019**
(21) Anmeldenummer: 13191164.6
(22) Anmeldetag: 31.10.2013
(51) Int. Cl.: A61B 3/00, A61B 3/12, A61B 3/13, G02B 7/02, G02B 21/24

(54) **Positioniereinheit und Beobachtungsvorrichtung**
Positioning unit and observation device
Unité de positionnement et dispositif d'observation

(30) Priorität: 02.11.2012 US 201213667488
(43) Veröffentlichungstag der Anmeldung: 07.05.2014
(73) Patentinhaber: Oculus Optikgeräte GmbH, 35582 Wetzlar (DE)
(72) Erfinder: Feiertag, Carsten, 35410 Hungen (DE); Pfeiffer, Günter, 35075 Gladenbach (DE); Kirchhübel, Rainer, 35614 Asslar (DE)
(74) Vertreter: advotec.

(56) Entgegenhaltungen:
- EP-A1- 2 478 832
- DE-A1-102008 011 608
- DE-U1- 9 415 219
- US-A1- 2006 198 026
- US-A1- 2007 091 256
- US-A1- 2007 188 699

## Beschreibung

Die Erfindung betrifft eine Positioniereinheit zur Positionierung einer zumindest ein optisches Element umfassenden optischen Einheit in einem Strahlengang eines Mikroskops zwischen einem Objektiv des Mikroskops und vor einem zu beobachtenden Auge, wobei die Positioniereinheit eine Anschlussvorrichtung umfasst, mittels der die Positioniereinheit an das Mikroskop koppelbar ist und wobei die Positioniereinheit eine Positioniervorrichtung umfasst, mittels der das optische Element relativ zum Mikroskop in Längsrichtung des Strahlengangs bewegbar ist, wobei die Positioniervorrichtung eine Einstelleinrichtung umfasst, mittels der eine Position des optischen Elements in Längsrichtung des Strahlengangs einstellbar ist, wobei die Einstelleinrichtung aus einer Gewindestange, einer Führungsstange und einem mit der Gewindestange und der Führungsstange verbundenen Halteelement gebildet ist, wobei die Positioniereinheit zu einem überwiegenden Teil aus Metall ausgebildet ist.

Mikroskope zur Durchführung von Augenoperationen werden regelmäßig für Operationen in einem vorderen Bereich eines Auges verwendet. Sollen auch in einem hinteren Bereich eines Auges derartige Eingriffe vorgenommen werden, ist es notwendig das Mikroskop mit einer Beobachtungsvorrichtung zu ergänzen, welche eine Fokussierung eben dieses Bereiches des Auges ermöglicht. Derartige Beobachtungsvorrichtungen umfassen zumindest eine Weitwinkellinse bzw. Ophthalmoskopierlinse zur Weitwinkelbetrachtung des betreffenden hinteren Teils des Auges, wobei die Ophthalmoskopierlinse ein Zwischenbild in einem Strahlengang vor einem Objektiv des Mikroskops zur Verfügung stellt, welches mit dem Mikroskop fokussiert werden kann. Zur Fokussierung des Zwischenbildes bedarf es einer Verkürzung einer Länge des Strahlengangs des Mikroskops, die durch die entsprechenden Einstelleinrichtungen am Mikroskop vorgenommen werden kann. Da während einer Augenoperation jedoch zwischen verschiedenen Betrachtungsweisen, mit und ohne Ophthalmoskopierlinse gewechselt werden muss, ist eine derartige Einstellung des Mikroskops hinderlich, sodass im Strahlengang vor dem Objektiv eine Reduzierlinse vorgesehen sein kann, die zur Verkürzung des Strahlengangs des Mikroskops dient und die gemeinsam mit der Ophthalmoskopierlinse verwendet wird. Die beiden Linsen werden von einer Positioniereinheit der Beobachtungsvorrichtung, welche unmittelbar am Mikroskop befestigt ist, gehaltert und können je nach Bedarf im Strahlengang positioniert werden, ohne dass es einer wesentlichen Anpassung des Mikroskops während einer Operation bedarf. Die Positioniereinheit umfasst regelmäßig eine Anschlussvorrichtung, mittels der die Positioniereinheit an das Mikroskop koppelbar ist. Weiter ist die Positioniereinheit so ausgebildet, dass die betreffenden Linsen einfach in den Strahlengang eingeschwenkt bzw. eingeschoben und wieder daraus entfernt werden können.

Um eine möglichst genaue Anpassung des Zwischenbildes der Ophthalmoskopierlinse an eine Brennweite des Mikroskopobjektivs vornehmen zu können, kann zumindest eine der Linsen längs des Strahlengangs des Mikroskops einstellbar ausgebildet sein. Bei bekannten Beobachtungsvorrichtungen ist zur längsverschiebbaren Einstellung der Linse beispielsweise an der Positioniereinheit eine lineare Führung ausgebildet, wobei die Linse mittels eines Einstellrades mit einem Gewindetrieb bewegt werden kann. Um während einer Operation eines Auge eine versehentliche Kollision der Ophthalmoskopierlinse mit dem Auge zu verhindern bzw. eine mögliche Augenverletzung zu vermeiden, ist die Positioniereinheit so ausgebildet, dass die Ophthalmoskopierlinse in Richtung des Objektivs des Mikroskops im Wesentlichen widerstandslos bewegbar ist, dass heißt bei einer Kollision mit dem Auge zurückweichen kann. Dies wird beispielsweise durch eine zweite lineare Führung erreicht, die ebenfalls eine Längsverschiebung der Ophthalmoskopierlinse ermöglicht.

Neben den zuvor beschriebenen, mechanischen und optischen Anforderungen ist es wichtig, dass die Beobachtungsvorrichtung bzw. die Positioniereinheit bei einer Operation im Wesentlichen steril ist, um eine mögliche Infektion eines Auges mit beispielsweise Keimen zu verhindern. Eine Infektionsgefahr besteht insbesondere dadurch, dass die Beobachtungsvorrichtung bei einer Operation relativ dicht an das betreffende Auge herangeführt wird. Es ist daher üblich, die betreffende Beobachtungsvorrichtung bzw. Positioniereinheit vor einer Operation durch beispielsweise Dampfsterilisation zu reinigen. Um eine wiederholte Sterilisation durchführen zu können, ist es zwingend notwendig alle Bauteile der Beobachtungsvorrichtung bzw. Positioniereinheit, mit Ausnahme eventuell vorhandener Dichtungen aus elastischen Materialien wie Gummi, aus Metall oder Glas auszubilden. Andere Werkstoffe, wie beispielsweise Kunststoffe, haben sich für eine wiederholte Sterilisation als nicht geeignet erwiesen. Auch die Linearführungen und der Gewindetrieb bedürfen zur Sicherstellung bestimmter Passungen einer maßhaltigen Ausbildung, sodass auch hier nur Bauteile aus Metall in Frage kommen. Um ein Eindringen von Wasser bei der Sterilisation in die Führungen zu verhindern, können diese mit Gummidichtungen oder Dichtungen aus anderen elastischen Materialien versehen sein. Die metallenen Bauteile werden regelmäßig aus Aluminium oder einer Aluminiumlegierung hergestellt. Aluminium ist besonders gut zerspanend bearbeitbar und weist ein geringes Gewicht auf, was für eine Handhabung der Beobachtungsvorrichtung von Vorteil ist. Die Bauteile aus Aluminium werden weiter mittels einer Eloxalschicht vor Korrosion geschützt.

Insbesondere wenn eine Beobachtungsvorrichtung bzw. eine Positioniereinheit relativ häufig sterilisiert wird, tritt das Problem auf, dass eine Aluminiumoberfläche in Folge chemischer Reaktionen angegriffen und zerstört wird. Ursächlich hierfür sind alkalische Reiniger, die zu einer Vorreinigung der Beobachtungsvorrichtung bzw. Positioniereinheit vor einer Dampfsterilisation verwendet werden. Die Beobachtungsvorrichtung bzw. Positioniereinheit ist daher aufgrund einer Zerstörung der Oberfläche der Bauteile aus Aluminium nur für eine begrenzte Anzahl Sterilisationszyklen einsetzbar und damit verwendbar. Angegriffene Oberflächen bzw. Bauteile aus Aluminium können nicht mehr in der erforderlichen Weise gereinigt und damit für Augenoperationen verwendet werden.

Die EP 2 478 832 A1 zeigt eine Positioniereinheit, die vollständig aus Kunststoff ausgebildet sein kann.

Die US 2007/188699 A1 betrifft eine Ophthalmoskopierlinse mit einem Halter für die Linse in verschiedenen Ausführungsformen. Insbesondere wird für die Ophthalmoskopierlinse ein Glas verwendet, welches autoklvierbar bzw. sterilisierbar ist. Gleichzeitig soll für das Material des Halters Titan verwendet werden, da dieses einen thermischen Ausdehnungskoeffizienten ähnlich dem des Glases aufweist. Damit soll eine entsprechende kraftschlüssige Fassung des Glases bzw. der Linse in dem Halter während des Autoklavierens sichergestellt werden.

Die DE 10 2008 011 608 beschreibt eine Positioniereinheit für eine Ophthalmoskopierlinse, wobei die Positioniereinheit im Wesentlichen aus zwei an einem Mikroskop verschwenkbaren Armen ausgebildet ist, und wobei an einem unteren Ende eines Armes zwei Ophthalmoskopierlinsen samt Halter drehbar, und damit in einen Strahlengang schwenkbar, angeordnet sind.

Die US 2007/091256 A1 offenbart, dass ein einstückiges Gehäuse bzw. eine Fassung zur Halterung einer Ophthalmoskopierlinse aus autoklavierbaren Materialien, wie Aluminium, rostfreiem Stahl, Titan oder einem Hochtemperaturpolymer ausgebildet sein kann.

Die DE 9415219 U1 betrifft eine Positioniereinheit mit den Merkmalen des Oberbegriffs des Anspruchs 1.

Der vorliegenden Erfindung liegt daher die Aufgabe zugrunde, eine Positioniereinheit und eine Beobachtungsvorrichtung vorzuschlagen, deren Verwendbarkeit nicht durch den üblichen Einsatz von Reinigungsmitteln beeinflusst ist.

Diese Aufgabe wird durch eine Positioniereinheit mit den Merkmalen des Anspruchs 1 und eine Beobachtungsvorrichtung mit den Merkmalen des Anspruchs 12 gelöst.

Die erfindungsgemäße Positioniereinheit zur Positionierung einer zumindest ein optisches Element umfassenden optischen Einheit in einem Strahlengang eines Mikroskops zwischen einem Objektiv des Mikroskops und vor einem zu beobachtenden Auge umfasst eine Anschlussvorrichtung, mittels der die Positioniereinheit an das Mikroskop koppelbar ist, wobei die Positioniereinheit weiter eine Positioniervorrichtung umfasst, mittels der das optische Element relativ zum Mikroskop in Längsrichtung des Strahlengangs bewegbar ist, wobei die Positioniervorrichtung eine Einstelleinrichtung umfasst, mittels der eine Position des optischen Elements in Längsrichtung des Strahlengangs einstellbar ist, wobei die Einstelleinrichtung aus einer Gewindestange, einer Führungsstange und einem mit der Gewindestange und der Führungsstange verbundenen Halteelement gebildet ist, wobei die Positioniereinheit zu einem überwiegenden Teil aus Metall ausgebildet ist, und wobei zumindest die Positioniervorrichtung frei von Aluminium ausgebildet ist, wobei eine Gleitflächenpaarung ausbildende, relativ zueinander bewegbare Bauteile der Positioniereinheit eine Materialpaarung von Titan/Stahl oder von Titan/Keramik aufweisen.

Folglich ist die Positioniereinheit zwar, mit Ausnahme erforderlicher Dichtungen aus Metall, ausgebildet, jedoch wird für Bauteile der Positioniereinheit bzw. der Positioniervorrichtung kein Aluminium oder eine Aluminiumlegierung verwendet. So ist sichergestellt, dass die bei Aluminium beobachtete Zerstörung einer Bauteiloberfläche durch eine alkalische Reinigung nicht erfolgen kann. Die Positioniereinheit kann dann nahezu beliebig oft einer alkalischen Reinigung bzw. Sterilisation unterzogen werden. Vorzugsweise werden zur Ausbildung der Positioniereinheit dann Metalle verwendet, die sich für eine alkalische Reinigung besonders gut eignen.

Vorteilhaft kann die Positioniereinheit aus Titan und Stahl oder Titan und Keramik ausgebildet sein. So kann sichergestellt werden, dass keine der verwendeten Werkstoffe durch eine alkalische Reinigung angegriffen werden. Weiter ist Titan hier als ein Werkstoff besonders gut geeignet, da Titan eine vergleichsweise geringe Dichte aufweist und somit auch bei einer zusätzlichen Verwendung von Stahl ein Gewicht der Positioniereinheit nicht wesentlich erhöht. Auch können einzelne Bauteile der Positioniereinheit aus einem keramischen Werkstoff ausgebildet sein. Keramische Werkstoffe können ebenfalls eine geringe Dichte und eine gute chemische Beständigkeit aufweisen. Alternativ ist es natürlich auch möglich die Positioniereinheit aus Titan, Stahl und Keramik auszubilden. Die Positioniereinheit kann dann vollständig aus den vorgenannten Werkstoffen ausgebildet sein.

Erfindungsgemäß weisen eine Gleitflächenpaarung ausbildende relativ zueinander bewegbare Bauteile der Positioniereinheit eine Materialpaarung von Titan/Stahl oder von Titan/Keramik auf. So können Materialpaarungen verwendet werden, die einen besonders günstigen Reibungskoeffizienten aufweisen. Gegebenenfalls kann dann sogar auf eine sonst übliche Schmierung der Gleitflächenpaarung verzichtet werden. Insbesondere Titan weist einen niedrigen Reibungskoeffizienten auf.

Weiter kann als Stahl ein rostfreier Stahl und als Titan eine Titanlegierung verwendet werden. Die Werkstoffe können dann noch besser an eine Verwendung angepasst werden. Dabei können der rostfreie Stahl und die Titanlegierung auch Aluminium als einen Legierungszusatz enthalten.

Erfindungsgemäß umfasst die Positioniervorrichtung eine Einstelleinrichtung, mittels der eine Position des optischen Elements in Längsrichtung des Strahlengangs einstellbar ist. Eine Bewegbarkeit des optischen Elements in Längsrichtung des Strahlengangs relativ zum Mikroskop ermöglicht eine Anpassung der optischen Einheit an das zu beobachtende Auge und/oder eine Anpassung des Strahlengangs des Mikroskops an ein im Strahlengang befindliches Zwischenbild, ohne dass diesbezügliche Einstellungen am Mikroskop vorgenommen werden müssten.

Erfindungsgemäß ist die Einstelleinrichtung aus einer Gewindestange, einer Führungsstange und einem mit der Gewindestange und der Führungsstange verbundenen Halteelement gebildet. Die Gewindestange und die Führungsstange können aus Stahl sowie das Halteelement aus Titan ausgebildet sein. Das Halteelement kann abschnittsweise Gewindegänge aufweisen, die in die Gewindestange eingreifen können. So ist es möglich, durch eine Drehung der Gewindestange das Halteelement entlang der Führungsstange zu verschieben und damit relativ zum Mikroskop in Längsrichtung des Strahlengangs zu bewegen bzw. genau einzustellen. Dabei muss das Halteelement die Gewindestange noch nicht einmal in Art einer Mutter umgeben. Weiter kann an der Gewindestange ein Rad zur Handbetätigung derselben ausgebildet sein. Eine Einstellung bzw. Positionierung des optischen Elements kann dann beispielsweise manuell durch eine Bedienperson erfolgen.

Zum Schutz eines Auges durch eine unbeabsichtigte Kollision mit dem optischen Element kann die Positioniervorrichtung eine Sicherheitseinrichtung aufweisen, die eine lose Bewegung des optischen Elements ermöglicht, wenn auf das optische Element eine Kraft in Richtung des Mikroskops ausgeübt wird. Das heißt, die Positioniereinrichtung bzw. Sicherheitseinrichtung kann so ausgebildet sein, dass bei einer Krafteinwirkung auf das optische Element, beispielsweise hervorgerufen durch eine Kollision mit dem betreffenden Auge, das optische Element im Wesentlichen widerstandslos in Richtung des Objektivs bewegt werden kann. Die Sicherheitseinrichtung kann so ausgebildet sein, dass die Positioniervorrichtung und die optische Einheit durch ihr jeweiliges Eigengewicht das optische Element in einer unteren Position in Nähe des Auges hält. Wird dann in Richtung des Mikroskops eine Kraft auf das optische Element aufgebracht, ist lediglich eine Gewichtskraft der optischen Einheit bzw. Positioniervorrichtung zu überwinden, um das optische Element bewegen zu können. Um eine unerwünschte Bewegung des optischen Elements auszuschließen, kann zu dessen Stabilisierung eine Feder an der Sicherheitseinrichtung vorgesehen werden, die eine zusätzliche Kraft in Richtung des Auges aufbringt.

Beispielsweise kann die Sicherheitseinrichtung aus einer in einem Halteelement geführten Haltestange gebildet sein, wobei die Haltestange aus Stahl oder Keramik und das Halteelement aus Titan ausgebildet sein kann. An einem unteren Ende der Haltestange kann dann ein optisches Element befestigt sein. Das Halteelement kann einstückig ausgebildet sein und auch ein Bestandteil einer Einstelleinrichtung sein.

Weiter kann die Positioniervorrichtung eine Halteeinrichtung umfassen, mittels der das optische Element in den Strahlengang hinein und heraus bewegbar ist und mittels der die Positioniervorrichtung mit der Anschlussvorrichtung werkzeuglos lösbar verbindbar sein kann. Die Anschlussvorrichtung kann unmittelbar mit dem Mikroskop fest verbindbar ausgebildet sein, wobei die Anschlussvorrichtung ohne zu Hilfenahme von zusätzlichem Werkzeug mit der Halteeinrichtung, beispielsweise in Art einer Steckverbindung, verbunden werden kann.

Weiter kann die Halteeinrichtung so ausgebildet sein, dass das optische Element in den Strahlengang einschiebbar oder einschwenkbar ist. Vorzugsweise kann das optische Element um eine quer zum Strahlengang verlaufende Achse zusammen mit der Positioniereinheit verschwenkt werden. So kann sichergestellt werden, dass die Positioniereinheit und die optische Einheit während einer Operation eine Sicht in einem Bewegungsraum des Operierenden außerhalb des betreffenden Auges nicht einschränkt bzw. behindert. Auch wird es so möglich, das optische Element je nach Bedarf einfach in den Strahlengang hinein und heraus zu bewegen.

So kann die Halteeinrichtung aus einem Haltearm zur Halterung einer Einstelleinrichtung und einem Befestigungselement zur Verbindung mit der Anschlussvorrichtung gebildet sein, wobei der Haltearm aus Titan und das Befestigungselement aus Stahl ausgebildet sein kann. Das Befestigungselement kann dann so ausgebildet sein, dass es besonders einfach mit der Anschlussvorrichtung verbunden oder beispielsweise verrastet werden kann.

Weiter kann das Befestigungselement eine Achse aufweisen, die mit dem Haltearm ein Drehgelenk ausbildet. Durch das Drehgelenk kann die Einstelleinrichtung relativ zur Anschlusseinrichtung verschwenkbar sein. Zur Ausbildung eines derartigen Drehgelenks werden nicht notwendigerweise zusätzliche Bauteile benötigt. Die Achse kann jedoch auch als ein einzelnes Bauteil aus Stahl ausgebildet sein, wobei der Haltearm aus Titan dann auf die Achse einfach aufgesteckt werden kann.

Zur einfacheren Handhabung kann das Drehgelenk zumindest eine Rasteinrichtung umfassen, mittels der das optische Element in einer Verwendungsposition im Strahlengang und/oder in einer Nichtverwendungsposition außerhalb des Strahlengangs arretierbar ist. Die Rasteinrichtung kann so ausgebildet sein, dass zwischen dem Haltearm und dem Befestigungselement eine Rastnase in Rastvertiefungen zum Eingriff der Rastnase ausgebildet ist. Die Rastnase und die Rastvertiefungen können jeweils an dem Haltearm oder dem Befestigungselement angeformt sein. Die Rastvertiefungen können dann so angeordnet sein, dass die Rastnase in der Verwendungsposition und der Nichtverwendungsposition jeweils in eine Rastvertiefung eingreift und so eine Arretierung des optischen Elements bzw. der Einstelleinrichtung ermöglicht.

Im Übrigen kann der Haltearm einer Haltefassung eine Reduzierlinse der optischen Einheit umfassen, wobei die Haltefassung aus Stahl ausgebildet sein kann. Die Haltefassung kann dann einfach in den Haltearm aus Titan eingesetzt werden. So ist es möglich die Reduzierlinse auch einfach gegen eine Reduzierlinse mit anderen optischen Eigenschaften auszuwechseln.

Die erfindungsgemäße Beobachtungsvorrichtung umfasst eine Positioniereinheit nach einem der Ansprüche 1 bis 11 und zumindest eine optische Einheit, wobei die optische Einheit zumindest ein optisches Element umfasst, dass als eine Ophthalmoskopierlinse ausgebildet ist, die zur Beobachtung eines Augenhintergrundes dient. Hinsichtlich der Vorteile der erfindungsgemäßen Beobachtungsvorrichtung wird auf die vorangehenden Vorteilsbeschreibungen der Positioniereinheit verwiesen.

Eine optische Einheit kann als ein weiteres optisches Element eine Reduzierlinse umfassen, die zur Anpassung des Strahlengangs dient. Weiter können auch zusätzliche, optische Einheiten zur Bildumkehr und/oder Vertauschen zweier Strahlengänge vorgesehen sein.

In einer Ausführungsform kann die Beobachtungsvorrichtung eine elektrische Antriebseinrichtung umfassen, die zur Bewegung des optischen Elements in Längsrichtung des Strahlengangs dient. Die elektrische Antriebseinrichtung kann in Art eines Elektromotors ausgebildet sein und eine gegebenenfalls vorhandene Einstelleinrichtung der Positioniereinheit antreiben. Der Elektromotor kann in einem separaten Gehäuse flüssigkeitsdicht aufgenommen sein und über ein Getriebe oder eine sonstige Kupplung mit der Positioniereinheit verbunden sein. So ist es auch möglich, den Elektromotor bzw. die elektrische Antriebseinrichtung zu sterilisieren. Vorzugsweise kann das Gehäuse aus Titan ausgebildet sein.

Weitere vorteilhafte Ausführungsformen der Beobachtungsvorrichtung ergeben sich aus den Merkmalsbeschreibungen der auf den Vorrichtungsanspruch 1 rückbezogenen Unteransprüche.

Nachfolgend werden bevorzugte Ausführungsformen der Erfindung unter Bezugnahme auf die beigefügten Zeichnungen näher erläutert.

Es zeigen:
- **Fig. 1**: Eine erste Ausführungsform einer Beobachtungsvorrichtung in einer perspektivischen Ansicht;
- **Fig. 2**: eine zweite Ausführungsform einer Beobachtungsvorrichtung in einer perspektivischen Ansicht;
- **Fig. 3**: eine dritte Ausführungsform einer Beobachtungsvorrichtung in einer perspektivischen Ansicht;
- **Fig. 4**: eine Anschlussvorrichtung in einer perspektivischen Ansicht.

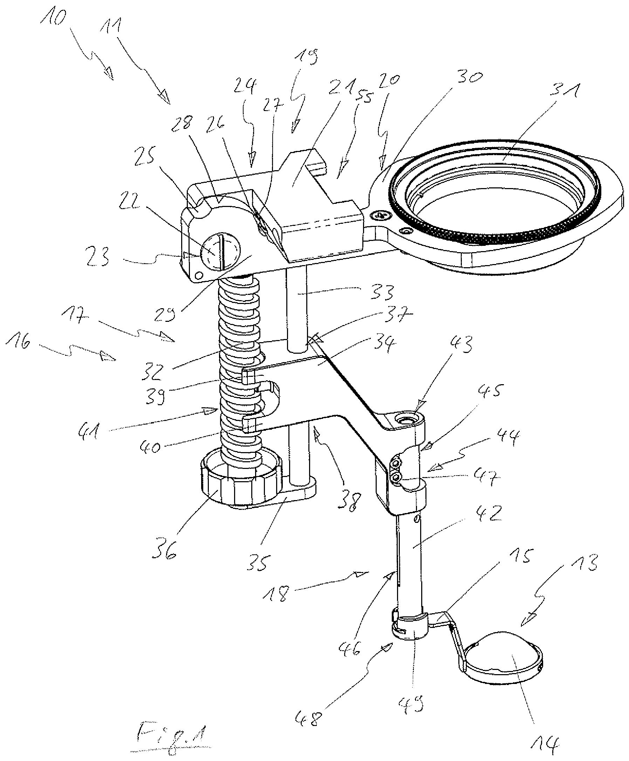

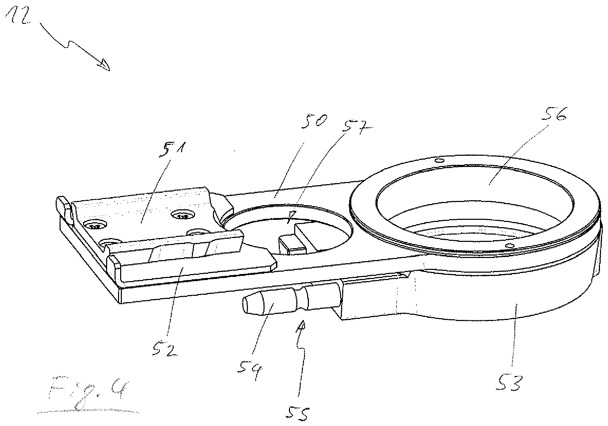

Die **Fig. 1** zeigt eine Beobachtungsvorrichtung 10 mit einer Positioniereinheit 11, jedoch ohne eine Anschlussvorrichtung. **Fig. 4** ist eine zugehörige Anschlussvorrichtung 12 zu entnehmen.

Die Beobachtungsvorrichtung 10 umfasst neben der Anschlussvorrichtung 12 und der Positioniereinheit 11 eine optische Einheit 13, von der hier eine Ophthalmoskopierlinse 14 an einem Haltearm 15 abgebildet ist. Die Positioniereinheit 11 umfasst weiter eine Positioniervorrichtung 16 mit einer Einstelleinrichtung 17, einer Sicherheitseinrichtung 18 und einer Halteeinrichtung 19. Die Halteeinrichtung 19 ist aus einem Haltearm 20 aus Titan zur Halterung der Einstelleinrichtung 17 und einem Befestigungselement 21 zur Verbindung mit der Anschlussvorrichtung 12 gebildet. Das Befestigungselement 21 besteht aus Stahl und weist eine Achse 22, ebenfalls aus Stahl, auf, die mit dem Haltearm 20 ein Drehgelenk 23 ausbildet.

Das Drehgelenk 23 umfasst eine Rasteinrichtung mittels der die Ophthalmoskopierlinse 14 in der hier gezeigten Verwendungsposition in einen nicht näher dargestellten Strahlengang oder in einer Nichtverwendungsposition außerhalb des Strahlengangs arretierbar ist. Die Rasteinrichtung 24 umfasst zwei Rastausnehmungen 25 und 26, den Haltearm 20 sowie eine Rastnase 27 am Befestigungselement 21. Die Rastnase 27 wird mittels einer hier nicht sichtbaren Feder, welche innerhalb des Befestigungselements 21 angeordnet ist, auf eine bogenförmige Anlagefläche 28 am Haltearm 20 gedrückt.

Der Haltearm 20 ist aus einem ersten Blech 29 und einem zweiten Blech 30 aus Titan ausgebildet, die miteinander verschraubt sind. In dem zweiten Blech 30 ist eine Haltefassung 31 aus Stahl für eine hier nicht dargestellte Reduzierlinse eingesetzt. An dem ersten Blech 29 ist eine Gewindestange 32 der Einstelleinrichtung 17 drehbar befestigt. Weiter ist an dem ersten Blech 29 eine Führungsstange 33 der Einstelleinrichtung 17 fest montiert. Die Gewindestange 32 und die Führungsstange 33 sind aus Stahl ausgebildet. Die Einstelleinrichtung 17 umfasst weiter ein Halteelement 34 und ein Lagerelement 35 aus Titan. Das Lagerelement 35 verbindet die Gewindestange 32 mit der Führungsstange 33, wobei die Gewindestange 32 drehbar in dem Lagerelement 35 gelagert ist. Im Übrigen ist an der Gewindestange 32 ein Betätigungsrad 36 angeordnet, mittels dem die Gewindestange 32 manuell gedreht werden kann. Das Halteelement 34 weist eine Durchgangsbohrung 37 auf, in die die Führungsstange 33 eingesetzt ist, sodass hier zwischen der Führungsstange 33 und dem Halteelement 34 eine Linearführung 38 ausgebildet ist. Weiter sind an dem Halteelement 34 eine obere und eine untere Eingriffsnase 39 bzw. 40 ausgebildet, die hier nicht sichtbare Gewindegänge zum übereinstimmenden Eingriff in ein Gewinde 41 der Gewindestange 32 aufweisen. Die Eingriffsnasen 39 und 40 umfassen dabei die Gewindestange 32 nur hälftig. Eine Drehung der Gewindestange 32 bewirkt demnach eine Bewegung des Halteelements 34 in Längsrichtung der Führungsstange 33 und damit in Längsrichtung des Strahlengangs.

An dem Halteelement 34 ist weiter eine Haltestange 42 befestigt, die zusammen mit dem Halteelement 34 die Sicherheitseinrichtung 18 ausbildet. Die Haltestange 42 besteht aus Stahl und ist in eine Durchgangsbohrung 43 im Halteelement 34 bewegbar eingesetzt. Demnach ist auch hier zwischen dem Halteelement 34 und der Haltestange 42 eine Linearführung 44 ausgebildet. Am Halteelement 34 ist weiter im Bereich der Durchgangsbohrung 43 eine Ausnehmung 45 ausgebildet, die die Durchgangsbohrung 43 abschnittsweise öffnet. An der Haltestange 42 ist eine Führungsnut 46 ausgebildet, in die ein Führungselement 47 am Halteelement 34 eingreift und so eine Längs- und Drehbewegung der Haltestange 42 relativ zum Halteelement 34 begrenzt. An einem unteren Ende 48 der Haltestange 42 ist ein Verbindungselement 49 angeordnet, in das der Haltearm 15 eingesteckt ist.

Die in **Fig. 4** gezeigte Anschlussvorrichtung 12 besteht aus einem Blech 50 aus Titan, an dem ein Führungselement 51 und ein Anschlagblech 52 aus Stahl zur Adaption der Anschlussvorrichtung 12 an einem hier nicht dargestellten Mikroskop fest montiert sind. Weiter ist an dem Blech 50 ein Halteelement 53 aus Titan befestigt, welches einen Raststift 54 aus Stahl zur Verbindung mit dem Halteelement 34 aufweist. Zwischen dem Halteelement 53 und dem Halteelement 34 ist somit eine übereinstimmende Steckverbindung 55 ausgebildet. In das Blech 50 und das Haltelement 53 ist weiter ein Ring 56 aus Stahl eingesetzt, der ein Objektiv eines hier nicht dargestellten Mikroskops umgeben kann. Zur Gewichtsreduktion ist in dem Blech 50 eine Ausnehmung 57 ausgebildet.

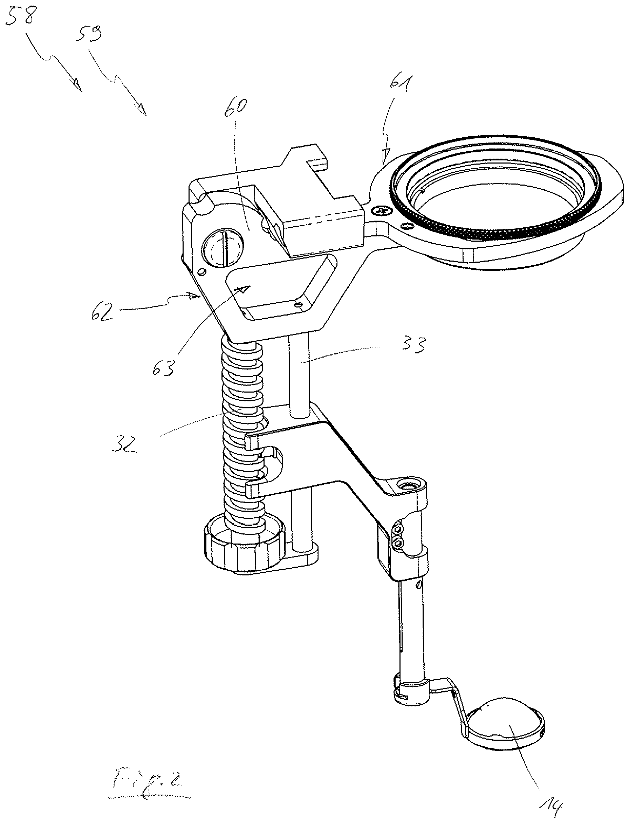

Die **Fig. 2** zeigt eine Beobachtungsvorrichtung 58 mit einer Positioniereinheit 59 ohne eine Anschlussvorrichtung. Die Beobachtungsvorrichtung 58 bzw. die Positioniereinheit 59 ist mit der Anschlussvorrichtung 12 aus **Fig. 4** verbindbar. Im Unterschied zur Positioniereinheit 11 aus **Fig. 1** ist bei der Positioniereinheit 59 ein erstes Blech 60 eines Haltearms 61 der Positioniereinheit 59 mit einer trapezförmigen Verlängerung 62 ausgebildet, um einen Abstand der Ophthalmoskopierlinse 40 gegenüber einem hier nicht dargestellten Objektiv eines Mikroskops zu vergrößern. In dem ersten Blech 60 ist weiter eine Ausnehmung 63 ausgebildet, um eine Gewichtsreduktion zu erzielen.

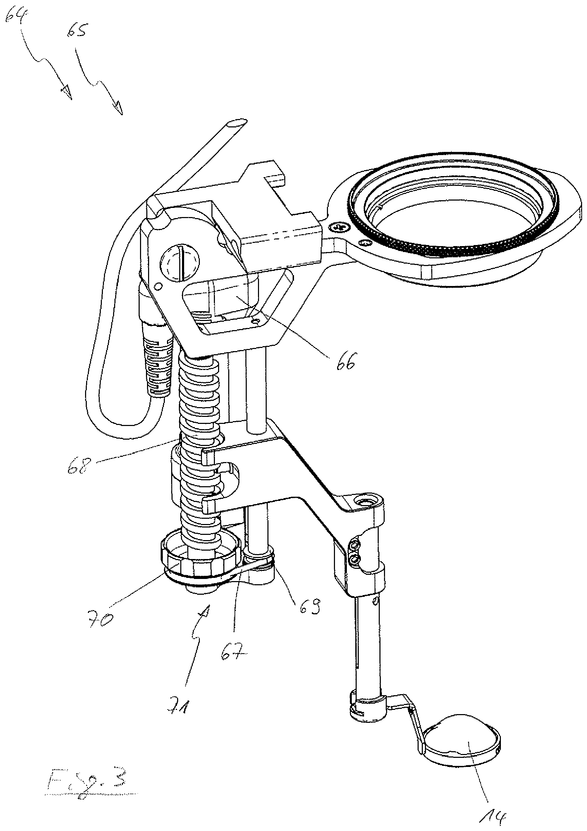

**Fig. 3** zeigt eine Ausführungsform einer Beobachtungsvorrichtung 64 mit einer Positioniereinheit 65 und ohne eine Anschlussvorrichtung, wobei auch hier die Beobachtungsvorrichtung 64 bzw. die Positioniereinheit 65 mit der Anschlussvorrichtung 12 aus **Fig. 4** verbindbar ist. Im Unterschied zu der in **Fig. 2** dargestellten Beobachtungsvorrichtung bzw. Positioniereinheit umfasst die Beobachtungsvorrichtung 64 einen elektromotorischen Antrieb 66, der an der Positioniereinheit 65 adaptiert ist. Der Antrieb 66 ist über einen Riemen 67 mit einer Gewindestange 68 und einer Umlenkrolle 69 verbunden. Die Umlenkrolle 69 ist an der Führungsstange 33 drehbar gelagert und an der Gewindestange 68 ist ein Betätigungsrad 70 abschnittsweise in Art einer Riemenscheibe ausgebildet, sodass der Riemen 67 das Betätigungsrad 70 antreiben und die Gewindestange 68 drehen kann. Der Antrieb 66 mit dem Riemen 67 und dem Betätigungsrad 70 bildet somit ein Riemengetriebe 71 aus.

## Patentansprüche

1. Positioniereinheit (11, 59, 65) zur Positionierung einer zumindest ein optisches Element umfassenden optischen Einheit in einem Strahlengang eines Mikroskops zwischen einem Objektiv des Mikroskops und vor einem zu beobachtenden Auge, wobei die Positioniereinheit eine Anschlussvorrichtung (12) umfasst, mittels der die Positioniereinheit an das Mikroskop koppelbar ist, und wobei die Positioniereinheit eine Positioniervorrichtung (16) umfasst, mittels der das optische Element relativ zum Mikroskop in Längsrichtung des Strahlengangs bewegbar ist, wobei die Positioniervorrichtung eine Einstelleinrichtung (17) umfasst, mittels der eine Position des optischen Elements in Längsrichtung des Strahlengangs einstellbar ist, wobei die Einstelleinrichtung aus einer Gewindestange (32, 68), einer Führungsstange (33) und einem mit der Gewindestange und der Führungsstange verbundenen Halteelement (34) gebildet ist, wobei die Positioniereinheit zu einem überwiegenden Teil aus Metall ausgebildet ist,
**dadurch gekennzeichnet,**
**dass** zumindest die Positioniervorrichtung frei von Aluminium ausgebildet ist, wobei eine Gleitflächenpaarung ausbildende, relativ zueinander bewegbare Bauteile der Positioniereinheit eine Materialpaarung von Titan/Stahl oder von Titan/Keramik aufweisen.

2. Positioniereinheit nach Anspruch 1,
**dadurch gekennzeichnet,**
**dass** die Positioniereinheit (11, 59, 65) aus Titan und Stahl oder Titan und Keramik ausgebildet ist.

3. Positioniereinheit nach Anspruch 1 oder 2,
**dadurch gekennzeichnet,**
**dass** als Stahl ein rostfreier Stahl und als Titan eine Titanlegierung verwendet wird.

4. Positioniereinheit nach einem der vorangehenden Ansprüche,
**dadurch gekennzeichnet,**
**dass** die Gewindestange und die Führungsstange aus Stahl und das Halteelement aus Titan ausgebildet sind.

5. Positioniereinheit nach einem der vorangehenden Ansprüche,
**dadurch gekennzeichnet,**
**dass** die Positioniervorrichtung (16) eine Sicherheitseinrichtung (18) aufweist, die eine lose Bewegung des optischen Elements ermöglicht, wenn auf das optische Element eine Kraft in Richtung des Mikroskops ausgeübt wird.

6. Positioniereinheit nach Anspruch 5,
**dadurch gekennzeichnet,**
**dass** die Sicherheitseinrichtung (18) aus einer in einem Halteelement (34) geführten Haltestange (42) gebildet ist, wobei die Haltestange aus Stahl oder Keramik und das Halteelement aus Titan ausgebildet ist.

7. Positioniereinheit nach einem der vorangehenden Ansprüche,
**dadurch gekennzeichnet,**
**dass** die Positioniervorrichtung (16) eine Halteeinrichtung (19) umfasst, mittels der das optische Element in den Strahlengang hinein und heraus bewegbar ist, und mittels der die Positioniervorrichtung mit der Anschlussvorrichtung (12) werkzeuglos lösbar verbindbar ist.

8. Positioniereinheit nach Anspruch 7,
**dadurch gekennzeichnet,**
**dass** die Halteeinrichtung (19) aus einem Haltearm (15, 61) zur Halterung einer Einstelleinrichtung (17) und einem Befestigungselement (21) zur Verbindung mit der Anschlussvorrichtung (12) gebildet ist, wobei der Haltearm aus Titan und das Befestigungselement aus Stahl ausgebildet ist.

9. Positioniereinheit nach Anspruch 8,
**dadurch gekennzeichnet,**
**dass** das Befestigungselement (21) eine Achse (22) aufweist, die mit dem Haltearm (15, 61) ein Drehgelenk (23) ausbildet.

10. Positioniereinheit nach Anspruch 9,
**dadurch gekennzeichnet,**
**dass** das Drehgelenk (23) zumindest eine Rasteinrichtung (24) umfasst, mittels der das optische Element in einer Verwendungsposition im Strahlengang und/oder in einer Nichtverwendungsposition außerhalb des Strahlengangs arretierbar ist.

11. Positioniereinheit nach einem der Ansprüche 8 bis 10,
**dadurch gekennzeichnet,**
**dass** der Haltearm (15, 61) eine Haltefassung (31) für eine Reduzierlinse der optischen Einheit umfasst, wobei die Haltefassung aus Stahl ausgebildet ist.

12. Beobachtungsvorrichtung (10, 58, 64) mit einer Positioniereinheit (11, 59, 65) nach einem der Ansprüche 1 bis 11 und zumindest einer optischen Einheit (13), wobei die optische Einheit zumindest ein optisches Element umfasst, das als eine Ophthalmoskopierlinse (14) ausgebildet ist, die zur Beobachtung eines Augenhintergrundes dient.

13. Beobachtungsvorrichtung nach Anspruch 12,
**dadurch gekennzeichnet,**
**dass** die optische Einheit (13) als ein weiteres optische Element eine Reduzierlinse umfasst, die zur Anpassung des Strahlengangs dient.

14. Beobachtungsvorrichtung nach Anspruch 12 oder 13,
**dadurch gekennzeichnet,**
**dass** die Beobachtungsvorrichtung (64) eine elektrische Antriebseinrichtung (66) umfasst, die zur Bewegung des optischen Elements in Längsrichtung des Strahlengangs dient.

## Claims

1. A positioning unit (11, 59, 65) for positioning an optical unit, which comprises at least one optical element, in an optical path of a microscope between a microscope lens and in front of an eye to be examined, the positioning unit comprising a connection device (12) by means of which the positioning unit can be coupled to the microscope, and the positioning unit comprising a positioning device (16) by means of which the optical element can be moved relative to the microscope in a longitudinal direction of the optical path, the positioning device comprising an adjusting mechanism (17) by means of which a position of the optical element can be adjusted in the longitudinal direction of the optical path, the adjusting mechanism being formed by a threaded rod (32, 68), a guiding rod (33) and a holding element (34) which is connected to the threaded rod and to the guiding rod, the positioning unit being predominantly made of metal,
**characterized in that**
at least the positioning device is free of aluminum, components of the positioning unit which form a sliding surface combination and are movable relative to one another having a material combination of titanium/steel or of titanium/ceramics.

2. The positioning unit according to claim 1,
**characterized in that**
the positioning unit (11, 59, 65) is made of titanium and steel or titanium and ceramics.

3. The positioning unit according to claim 1 or 2,
**characterized in that**
a stainless steel is used as steel and a titanium alloy is used as titanium.

4. The positioning unit according to any one of the preceding claims,
**characterized in that**
the threaded rod and the guiding rod are made of steel and the holding element is made of titanium.

5. The positioning unit according to any one of the preceding claims,
**characterized in that**
the positioning device (16) comprises a safety mechanism (18) which allows a lose movement of the optical element when a force is applied to the optical element in the direction of the microscope.

6. The positioning unit according to claim 5,
**characterized in that**
the safety mechanism (18) is formed by a holding rod (42) guided in a holding element (34), the holding rod being made of steel or ceramics and the holding element being made of titanium.

7. The positioning unit according to any one of the preceding claims,
**characterized in that**
the positioning device (16) comprises a holding mechanism (19) by means of which the optical element can be moved in and out of the optical path and by means of which the positioning unit can be detachably connected to the connection device (12) without tools.

8. The positioning unit according to claim 7,
**characterized in that**
the holding mechanism (19) is formed by a holding arm (15, 61) for holding an adjusting mechanism (17) and a fastening element (21) for the connection to the connection device (12), the holding arm being made of titanium and the fastening element being made of steel.

9. The positioning unit according to claim 8,
**characterized in that**
the fastening element (21) has an axis (22) which, together with the holding arm (15, 61), forms a swivel joint (23).

10. The positioning unit according to claim 9,
**characterized in that**
the swivel joint (23) comprises at least one locking mechanism (24) by means of which the optical element can be locked in a usage position in the optical path and/or in a non-usage position outside the optical path.

11. The positioning unit according to any one of claims 8 to 10,
**characterized in that**
the holding arm (15, 61) comprises a holding socket (31) for a reducing lens of the optical unit, the holding socket being made of steel.

12. An examination apparatus (10, 58, 64) comprising a positioning unit (11, 59, 65) according to any one of claims 1 to 11 and at least one optical unit (13), the optical unit comprising at least one optical element which is realized as an ophthalmoscopic lens (14), which is used to examine an ocular fundus.

13. The examination apparatus according to claim 12,
**characterized in that**
the optical unit (13) comprises a reducing lens as a further optical element, which is used to adjust the optical path.

14. The examination apparatus according to claim 12 or 13,
**characterized in that**
the examination apparatus (64) comprises an electric drive (66), which is used to move the optical element in the longitudinal direction of the optical path.

## Revendications

1. Unité de positionnement (11, 59, 65) destinée à positionner une unité optique, qui comprend au moins un élément optique, dans un trajet optique d'un microscope entre un objectif du microscope et devant un oeil à examiner, l'unité de positionnement comprenant un dispositif de raccordement (12) par lequel l'unité de positionnement peut être couplée au microscope, et l'unité de positionnement comprenant un dispositif de positionnement (16) par lequel l'élément optique peut être déplacé par rapport au microscope dans une direction longitudinale du trajet optique, le dispositif de positionnement comprenant un mécanisme d'ajustement (17) par lequel une position de l'élément optique peut être ajustée dans la direction longitudinale du trajet optique, le mécanisme d'ajustement étant formé par une tige filetée (32, 68), une tige de guidage (33) et un élément de support (34) qui est relié avec la tige filetée et avec la tige de guidage, l'unité de positionnement étant principalement en métal,
**caractérisée en ce**
**qu'**au moins le dispositif de positionnement est sans aluminium, des composants de l'unité de positionnement qui forment une paire de surfaces de glissement et qui sont mobiles l'un par rapport à l'autre ayant une combinaison de matériaux de titane/acier ou de titane/céramique.

2. Unité de positionnement selon la revendication 1,
**caractérisée en ce que**
l'unité de positionnement (11, 59, 65) est en titane et acier ou en titane et céramique.

3. Unité de positionnement selon la revendication 1 ou 2,
**caractérisée en ce**
**qu'**un acier inoxydable et utilisé comme acier et un alliage de titane est utilisé comme titane.

4. Unité de positionnement selon l'une quelconque des revendications précédentes,
**caractérisée en ce que**
la tige filetée et la tige de guidage sont en acier et l'élément de support est en titane.

5. Unité de positionnement selon l'une quelconque des revendications précédentes,
**caractérisée en ce que**
le dispositif de positionnement (16) comprend un mécanisme de sécurité (18) qui permet à l'élément optique de se déplacer de manière desserrée quand une force est appliquée à l'élément optique dans la direction du microscope.

6. Unité de positionnement selon la revendication 5,
**caractérisée en ce que**
le mécanisme de sécurité (18) est formé par une tige de support (42) guidée dans un élément de support (34), la tige de support étant en acier ou céramique et l'élément de support étant en titane.

7. Unité de positionnement selon l'une quelconque des revendications précédentes,
**caractérisée en ce que**
le dispositif de positionnement (16) comprend un mécanisme de support (19) par lequel l'élément optique peut être introduit dans le trajet optique et sorti du trajet optique et par lequel l'unité de positionnement peut être reliée de manière détachable avec le dispositif de raccordement (12) sans outils.

8. Unité de positionnement selon la revendication 7,
**caractérisée en ce que**
le mécanisme de support (19) est formé par un bras de support (15, 61) destiné à supporter un mécanisme d'ajustement (17) et par un élément de fixation (21) pour la liaison au dispositif de raccordement (12), le bras de support étant en titane et l'élément de fixation étant en acier.

9. Unité de positionnement selon la revendication 8,
**caractérisée en ce que**
l'élément de fixation (21) a un axe (22) qui forme une articulation tournante (23) avec le bras de support (15, 61).

10. Unité de positionnement selon la revendication 9,
**caractérisée en ce que**
l'articulation tournante (23) comprend au moins un mécanisme d'arrêt (24) par lequel l'élément optique peut être arrêté dans une position d'utilisation dans le trajet optique et/ou dans une position de non-utilisation hors du trajet optique.

11. Unité de positionnement selon l'une quelconque des revendications 8 à 10,
**caractérisée en ce que**
le bras de support (15, 61) comprend une monture de support (31) pour une lentille de réduction de l'unité optique, la monture de support étant en acier.

12. Dispositif d'examen (10, 58, 64) comprenant une unité de positionnement (11, 59, 65) selon l'une quelconque des revendications 1 à 11 et au moins une unité optique (13), l'unité optique comprenant au moins un élément optique qui est réalisé comme lentille ophtalmoscopique (14), qui sert à examiner un fond de l'oeil.

13. Dispositif d'examen selon la revendication 12,
**caractérisé en ce que**
l'unité optique (13) comprend une lentille de réduction comme un autre élément optique, qui sert à ajuster le trajet optique.

14. Dispositif d'examen selon la revendication 12 ou 13,
**caractérisé en ce que**
le dispositif d'examen (64) comprend un mécanisme d'entraînement électrique (66) qui sert à déplacer l'élément optique dans la direction longitudinale du trajet optique.
